# EUROPÄISCHE PATENTANMELDUNG

(11) **EP 2 078 521 A1**
(43) Veröffentlichungstag der Anmeldung: **15.07.2009**
(21) Anmeldenummer: 08000265.2
(22) Anmeldetag: 08.01.2008
(51) Int. Cl.: A61K 8/35, A61K 8/49, A61Q 17/04

(54) **Kosmetische Zusammensetzung, enthaltend ein Benzotriazolderivat und einen AHR-Antagonisten**

(71) Anmelder: STADA ARZNEIMITTEL AG, 61118 Bad Vilbel (DE)
(72) Erfinder: Hansen, Peter, Dr., 63303 Dreieich (DE); Heppner, Andrea, 61197 Florstadt (DE); Rillmann, Thomas, Dr., 76756 Bellheim (DE); Schumann, Cristof, 35767 Breitscheid-Erdbach (DE)
(74) Vertreter: Hamm, Volker

(57) **Zusammenfassung**

Die Erfindung betrifft kosmetische Zusammensetzungen zum Schutz der Haut und/oder Haare gegen UV-Strahlung, enthaltend ein Benzotriazolderivat und einen AHR-Antagonisten.

## Beschreibung

Die Erfindung betrifft neue kosmetische Zusammensetzungen zur topischen Anwendung, die zum Lichtschutz der Haut und/oder der Haare gegen Ultraviolett-Strahlung (UV-Strahlung) vorgesehen sind.

Die schädigende Wirkung des ultravioletten Teils der Sonnenstrahlung auf die Haut ist seit langem bekannt. Derartige Schädigungen können eine einfache Hautreizung, z.B. ein leichter Sonnenbrand sein, können sich aber auch in Zellschädigungen manifestieren. Es ist bekannt, dass vor allem durch UV-B-Strahlung (das ist Strahlung mit einer Wellenlänge zwischen 290 nm und 320 nm) in der Haut DNS-Schäden hervorgerufen werden können, die zu Zellmutationen und somit zu Hautkrebs führen.

Auch werden die vorzeitige Hautalterung, die Bildung von Falten und die Erschlaffung des Hautbindegewebes durch UV-Strahlung beschleunigt; verantwortlich hierfür ist vor allem die langwellige UV-A-Strahlung (das ist Strahlung mit einer Wellenlänge zwischen 320 nm und 400 nm), Auch sie begünstigt die Auslösung einer Erythernbildung oder verstärkt diese Reaktion bei manchen Personen, und sie kann sogar die Ursache für durch Licht ausgelöste toxische oder allergische Reaktionen sein.

Zur Vorbeugung gegen derartige Schädigungen sind in den letzten Jahren immer effektivere Sonnenschutzmittel entwickelt worden, in denen zahlreiche physikalische und chemische Filtersubstanzen die Haut vor UV-A-Stralung und vor UV-B-Strahlung schützen sollen.

Physikalische Lichtschutzfilter, wie z.B. Oxide von Metallen, wirken auf der Haut verteilt wie kleine Spiegel, die die UV-Strahlung reflektieren und streuen.

Durch chemische Lichtschutzfilter wird die auf die Haut auftreffende UV-Strahlung in Wärmeenergie umgewandelt. Chemische Lichtschutzfilter können sowohl lipophide als auch hydrophile Eigenschaften aufweisen und entsprechend dieser Eigenschaften unterscheidet man zwischen öllöslichen und wasserlöslichen Filtern.

Lichtschutzzubereitungen werden oftmals als Emulsionen, Lotionen oder Gele formuliert.

Aufgabe der vorliegenden Erfindung war es, gegenüber dem Stand der Technik verbesserte Lichtschutzzusammensetzungen anzugeben.

Durch umfangreiche Untersuchungen hat die Anmelderin in unerwarteter und überraschender Weise festgestellt, dass eine Kombination von zwei bereits im Stand der Technik bekannten Verbindungen aufgrund einer synergistischen Wirkung die Erzielung von Sonnenschutzzusammensetzungen erlaubt, die einen deutlich verbesserten UV-Schutz aufweisen, der in allen Fällen höher ist als der, der bei gleicher Konzentration der Verbindungen und identischem Träger unter alleiniger Verwendung der einen oder der anderen Verbindung erhalten wird.

Bei der einen Verbindung handelt es sich um einen UV-Filter aus der Gruppe der Benzotriazolderivate der Formel 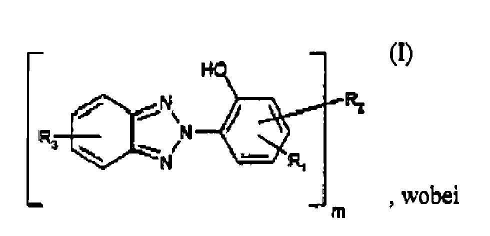
- R₁: C₁-C₃₀-Alkyl, C₁-C₅-Alkoxy, C₁-C₅-Alkoxycarbonyl, C₅-C₇-Cycloalkyl, C₆-C₁₀-Aryl, Arylalkyl, -SO₃M oder ein Radikal der Formel 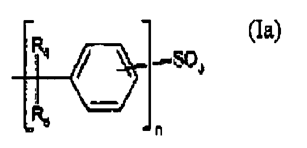 ist;
- R₃: Wasserstoff, C₁-C₅-Alkyl, C₁-C₅-Alkoxy, Halogen, vorzugsweise Cl oder Hydroxy ist;
- R₄ und R₅: voneinander unabhängig Wasserstoff oder C₁-C₅-Alkyl sind;
- m: 1 oder 2 ist;
- n: 0 oder 1 ist;
wenn m =1 ist, R₂ Wasserstoff, unsubstituiertes oder phenylsubstituiertes
C₁-C₂₁-Alkyl oder C₆-C₁₀-Aryl ist;
wenn m = 2 ist, R₂ eine Bindung oder -(CH₂)ₚ ist und p 1, 2 oder 3 ist,
wobei bevorzugt ein Benzotriazolderivat der Formel 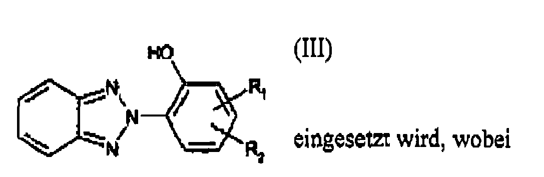
- R₁: C₁-C₃₀-Alkyl ist; und
- R₂: Wasserstoff oder C₁-C₁₂-Alkyl ist
und wobei besonders bevorzugt ein Benzotriazolderivat der Formel 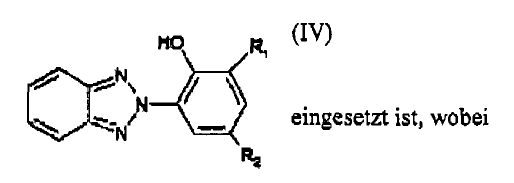
- R₁: eine Mischung aus wenigstens drei isomeren, verzweigten sekundären Alkylgruppen mit je 8-30 Kohlenstoffatomen der Formel 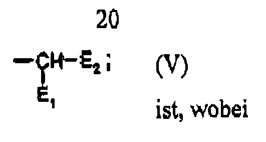
- E₁: geradkettiges C₁-C₁₄-Alkyl ist;
- E₂: geradkettiges C₄-C₁₅-Alkyl ist;
wobei die Gesamtzahl an Kohlenstoffatomen in E₁ plus E₂ 7 bis 29 beträgt; und
- R₂: C₁-C₅-Alkyl ist.

Ebenfalls besonders bevorzugt ist R₁ = C₈-C₁₆-Alkyl und insbesondere bevorzugt ist das Benzotriazolderivat der Formel

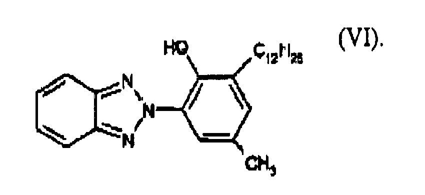

Bei der anderen Verbindung ii) handelt es sich um eine Verbindung der Formel 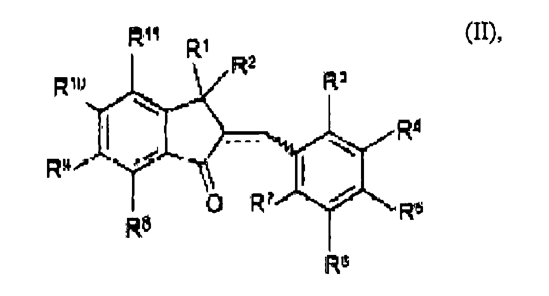 wobei
- R¹ und R²: unabhängig voneinander Wasserstoff oder C₁-C₁₂-Alkyl sind;
- R³ bis R¹¹: unabhängig voneinander Wasserstoff, C₁-C₁₂-Alkyl, Hydroxy oder C₁-C₁₂-Alkoxy sind
und die gestrichelte Linie eine Doppelbindung oder zwei Wasserstoffatome darstellt,
wobei bevorzugt Verbindungen der Formel IIa, IIb, IIc und IId eingesetzt werden:

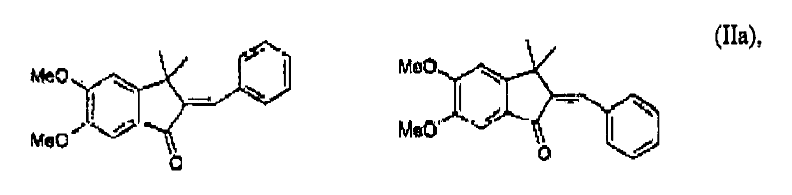

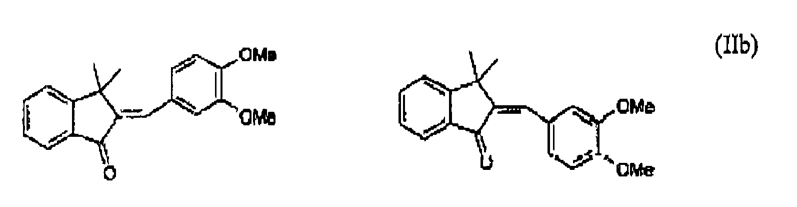

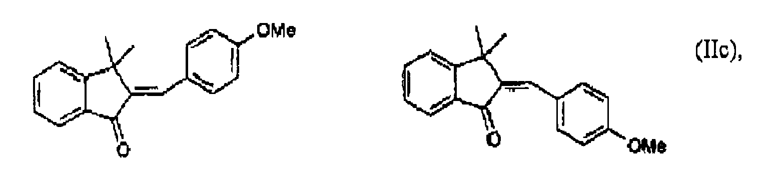

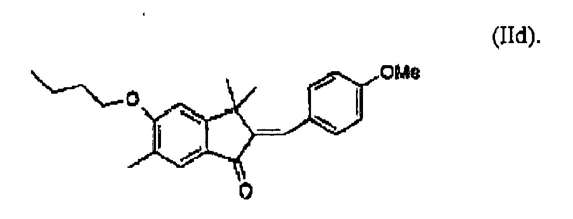

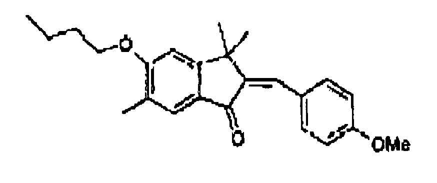

Die Benzotriazolderivate gemäß den Formeln I, III, IV und VI weisen UVabsorbierende Eigenschaften auf. Sie werden beispielsweise in der internationalen Anmeldung WO 2006/114381 beschrieben. Sie sind in den fertigen Formulierungen vorzugsweise in einer Menge von 0,1 bis 20 Gew.-% enthalten.

Die Benzotriazolderivate gemäß vorliegender Erfindung können in mikronisierter Form in den erfindungsgemäßen Zusammensetzungen vorliegen. Die bevorzugte mittlere Partikelgröße liegt dann im Bereich von 0,01 µm bis 2 µm. Sie können gemäß im Stand der Technik bekannten Verfahren mikronisiert werden. Unter anderem können sie in Gegenwart von Mahlhilfsmitteln mikronisiert werden. Als Mahlhilfsmittel können beispielsweise Alkylpolyglucoside verwendet werden.

Die Verbindungen der Formel II bzw. IIa, IIb, IIc und IId sind sogenannte Aryl-Hydrocarbon-Rezeptor-Antagonisten (Ah-Rezeptor-Antagonist),

Die Wirkweise von Aryl-Hydrocarbon-Rezeptor-Antagonisten und die Herstellung der Aryl-Hydrocarbon-Rezeptor-Antagonisten gemäß Formel II, IIa, IIb, IIc und IId ist in WO 2007/128723 beschrieben, auf die hier Bezug genommen wird.

UV-B-Strahlung kann in der Haut beispielsweise durch Bildung von Cyclobutan-Pyrimidin-Dimeren, durch Aktivierung von Membranproteinen, wie den Epidermal-Growth-Faktor-Rezeptor (EGFR) oder durch Induktion des Cytochroms P450 (CYP) IAI eine Photocarcinogenese auslösen, zu vorzeitiger Photoalterung sowie einer Immunsuppression fuhren.

Die Aryl-Hydrocarbon-Rezeptoren kommen in zahlreichen Geweben und Zellen vor. Sie spielen eine wichtige Rolle in der Entgiftung von exogenen Fremdstoffen und durch W-H-Hesrrahlung gebildeten Photoprodukten, wie beispielsweise dem Aryl-Hydrocarbon-Rezeptor-Liganden Formylindol (2,3)Carbazol (FICZ). Die Aktivierung der Aryl-Hydrocarbon-Rezeptoren stellt aber auch den ersten Schritt in der durch UV-B-Strahlung induzierten Stimulation des Epidermal-Growth-Faktor-Rezeptors dar. Der durch Aktivierung des Aryl-Hydrocarbon-Rezeptors gebildete Rezeptor-Liganden-Komplex kann beispielsweise durch Bindung an die DNA im Zellkern die Produktion von Enzymen der Cytochrom P450-Familie aktivieren, die wiederum in der Photocarcinogenese eine Rolle spielen.

Damit führt die durch UV-B-Strahlung induzierte Aryl-Hydrocarbon-Rezeptor-Aktivierung nebst der Ausbildung von oxidativern Stress zur Bildung von Photocarcinogenese, Hautirritation, atopischer Dermatitis und Hautalterung, Die Verwendung von Antagonisten des Aryl-Hydrocarbon-Rezeptors führt damit zu einem verbesserten Schutz der Haut vor UV-Strahlung, insbesondere den negativen Auswirkungen einer durch UV-B-Strahlung induzierten Aktivierung von Aryl-Hydrocarbon-Rezeptoren, die wiederum in der Erzeugung von oxidativem Stress, der Entstehung von Krebs, der Auslösung von Hautirritationen, dem Entstehen einer atopischen Dermatitis und der vorzeitigen Hautalterung bestehen.

Die Verbindungen der Formel II, IIa, IIb, IIc und IId liegen in den erfindungsgemäßen Zusammensetzungen bevorzugt in Mengen von 0,0001 bis 10 Gew.-%, besonders bevorzugt in Mengen von 0,001 bis 5 Gew.-% und insbesondere 0,01 bis 1 Gew.-% vor. Insbesondere bevorzugt ist die Verbindung gemäß Formel IIa.

Vorzugsweise werden die Benzotriazolderivate gemäß Merkmal i) und die Aryl-Hydrocarbon-Rezeptor-Antagonisten gemäß Merkmal ii) in Mengenanteilen eingesetzt, die so ausgewählt sind, dass der synergistische Effekt hinsichtlich des durch die resultierende Kombination erzielten Schutzes der Haut vor UV-Strahlung, insbesondere UV-B-Strahlung, optimal ist.

Die erfindungsgemäßen kosmetischen Zusammensetzungen können des weiteren übliche Zusatzstoffe für kosmetische Zusammensetzungen, wie z.B. Verdickungsmittel, Glättungsmittel, anfeuchtende und/oder feuchthaltende Substanzen, oberflächenaktive Mittel, Entschäumer, Fette, Öle, Wachse, Bakterizide, Treibmittel, Farbstoffe und/oder Pigmente oder dergleichen umfassen.

Die erfindungsgemäßen kosmetischen Zusammensetzungen umfassen in bevorzugten Ausführungsformen der Erfindung des weiteren einen oder mehrere anorganische und/oder organische UV-Filter.

Vorteilhafte anorganische UV-Filter sind Metalloxide, insbesondere Oxide des Titans oder Zinks. Wenn anorganische Pigmente enthalten sind, werden diese vorzugsweise in mikronisierter Form eingesetzt, vorzugsweise in Teilchengrößen < 100 µm, Sie sind dann bevorzugt in Mengen von 1 bis 25 Gew.-%, inbesondere 1 bis 10 Gew.-% enthalten. Dabei ist es besonders vorteilhaft, wenn die physikalischen Lichtschutzfilter in hydrophober Fort vorliegen, d.h., dass sie oberflächig wasserabweisend ausgestaltet sind. Dies kann z.B. dadurch erfolgen, dass die Pigmentteilchen mit einer hydrophoben Oberflächenschicht aus Silikon überzogen werden. Derartige Pigmente sind kommerziell erhältlich beispielsweise von der Firma Tayca unter der Handelsbezeichnung MT100T.

Erfindungsgemäße organische UV-Filter sind öllösliche oder wasserlösliche UV-A- und/oder UV-B-Filter. Dabei sind als geeignete organische UV-Filter insbesondere die folgenden zu nennen:
p-Aminobenzoesäurederivate, z.B. 4-Aminobenzoesäure, 4-Dimethylaminobenzoesäure-2-ethylhexylester (Eusolex 6007) oder ethoxyliertes Ethyl-4-aminobenzoat (Uvinul P25), Salicylsäurederivate, z.B. 3,3,5-Trimethyl-cyclohexyl-salicylat (Neo Heliopan HMS) oder Salicylsäure-2-ethylhexylester (Neo Heliopan OS),
Benzophenonderivate, z.B. 2-Hydroxy-4-methoxy-benzophenon (Neo Heliopan BB), 2-Hydroxy-4-methoxy-benzophenon-5-sulfonsäure und ihr Natriumsalz (Uvinul MS 40) oder 2-[4-(Diethylamino)-2-hydroxybenzoyl]-Benzoesäurehexylester (Uvinul A Plus),
Sulfonsäurederivate, z.B. 2-Phenylbenzimidazol-5-sulfonsäure und ihre Kalium-, Natrium- und Triethanolaminsalze (Neo Heliopan Hydro) oder Benzol-1,4-di(3-mathyliden-10-camphersulfonsäure) (Mexoryl SX),
Dibenzoylmethanderivate, z.B. 1-(4-tert-Butylphenyl)-3-(4-methoxyphenyl)propan-1,3-dion (Parsol 1789),
Diphenylacrylate, z.B. 2-Cyano-3,3-diphenylacrylsäure-2-ethylhexylester (Neo Heliopan 303),
Methoxyzimtsäurederivate, z.B. 4-Methoxyzimtsäure-2-ethylhexylester (Neo Heliopan AV) oder 4-Methoxy-zimtsäureisoamylester (Neo Heliopan E 1000),
Campherderivate, z.B. 3-(4'-Trimethylammonium)-benzyliden-boman-2-on-methylsulfat (Mexoryl SO), 3-(4'-Sulfo)-benzyliden-bornan-2-on und seine Salze (Mexoryl SX) oder 3-(4'-Methylbenzyliden)-DL-campher (Eusolex 6300) oder 3-Benzylidencampher (Mexoryl SDS20),
Triazinderivate, z.B. 2,4,6-Tris[p-(2-ethylhexyl-oxycarbonyl)anilino] 1,3,5-triazin (Uvinul T 150), 4,4'-[(6-[4-((1,1-Dimethylethyl)aminocarbonyl)phenyl-amino]-1,3,5-triazin-2,4-diyl)diimino]bis(benzoesäure-2-ethylhexylester) (Uvasorb HEB) oder 2,4-Bis[4-(2-ethylhexyloxy)-2-hydroxyphenyl]-6-(4-methoxyphenyl)-1,3,5-triazin (Tinosorb S),
Benzotriazolderivate, z.B. 2,2'-Methylen-bis(6-(2H-benzotriazol-2-yl)-4-(1,1,3,3-tetramethylbutyl)phenol) (Tinosorb M) oder 2-(2H-Benzotriazol-2-yl)-4-methyl-6-(2-methyl-3-(1,3,3,3-tetramethyl-1-(trimethylsilyloxy) disiloxanyl)propyl)phenol (Mexoryl XL),
Henzimidazolderivate, z.B. 2,2' -(1,4.Phenylen)bis(1H-benzimidazol-4,6-disulfonsäure, Mononatriumsalz) (Neo Heliopan AP),
Benzoxazol-Derivate, z.B. 2,4-Bis-[5-1(dimethylpropyl)benzoxazol-2-yl-(4-phenyl)-imino]-6-(2-ethylhexyl)-imino-1,3,5-triazin (Uvasorb K2A),
Polymer von N-[2(und 4)-(2-oxoborn-3-ylidenmethyl)benzyl]acrylamid (Mexoryl SW),
3-(4-(2,2-bis Ethoxycarbonylvinyl)-phenoxy)propenyl)-methoxysiloxan/Dimethylsiloxan-copolymer (Parsol SLX).

Die organischen UV-Filter sind, sofern enthalten, bevorzugt in Mengen von 0,1 bis 15 Gew.-%, vorzugsweise 0,5 bis 10 Gew.-%, insbesondere 0,5 bis 8 Gew.-% in den erfindungsgemäßen Zusammensetzungen enthalten.

Die erfindungsgemäßen Lichtschutzzubereitungen können in üblicher Weise formuliert werden, z.B. als O/W-Emulsion, W/O-Emulsion, Hydrogel, Hydrodispersion oder auch in Form von wasserfreien Produkten.

Die O/W- bzw. W/O-Emulsionen umfassen grundsätzlich eine Ölphase, Wasser, ggf. Alkohol sowie die erfindungsgemäße Wirkstoffkombination. Die Ölphase kann dabei aus Fetten, Ölen, gelösten Wachsen oder sonstigen lipophilen Bestandteilen gebildet werden, insbesondere auch öllöslichen UV-Filtern. Als Öle können vorteilhaft Paraffinöl, mittelkettige Triglyceride, wie beispielsweise Myritol 318, Octyldodecanol, Isopropylmyristat, Jojobaöl, Kokosnussöl oder Rhizinusöl enthalten sein. Die wässrige Phase der erfindungsgemäßen Emulsionen wird üblicherweise aus Wasser, ggf, im Gemisch mit Alkohol gebildet.

Die erfindungsgemäßen Hydrodispersionen umfassen grundsätzlich eine Ölphase, ein geeignetes Verdickungsmittel, Wasser sowie einen wirksamen Gehalt an der erfindungsgemäßen Wirkstoffkombination. Die Ölphase kann wiederum aus Fetten, Ölen, gelösten Wachsen oder anderen lipophilen Bestandteilen gebildet werden, wobei als Öle vorteilhaft die oben genannten Öle enthalten sein können.

Die Hydrogele gemäß der Erfindung umfassen neben der erfindungsgemäßen Wirkstoffkombination grundsätzlich Wasser, ein geeignetes Verdickungsmittel sowie ggf. Alkohol.

Als Verdickungsmittel kommen für die Hydrodispersionsgele und Hydrogele alle üblicherweise verwendeten Verdickungsmittel in Betracht, bevorzugt werden jedoch Polyacrylsäurederivate, wie beispielsweise Polyacrylate aus der Gruppe der sogenannten Carbopole, Acrylsäurecopolymere, wie beispielsweise Pemulen-TR-1 oder Xanthan. Der Gehalt an Verdickungsmitteln in den erfindungsgemäßen Gelen beträgt 0,05 bis 20 Gew,-%, bevorzugt 0,1 bis 3 Gew.-% und besonders bevorzugt 0,5 bis 1 Gew.-%.

Besonders bevorzugt sind die erfindungsgemäßen Zusammensetzungen frei von die Hautverträglichkeit potentiell negativ beeinflussenden Zusatzstoffen, insbesondere enthalten sie keine PEG-Ernulgatoren, Konservierungsstoffe oder Duftstoffe. Vorzugsweise sind die Zubereitungen wasserfest bzw. seewasserfest.

Die folgenden Ausführungsbeispiele veranschaulichen erfindungsgemäße Zusammensetzungen, wobei die Inhaltsstoffe gemäß der INCI-Nomenklatur bezeichnet werden. Sie sollen die Erfindung nicht beschränken.

### I. O/W-Emulsionen:

| Inhaltsstoffe | Gehalt [Gew.-%] | | | | | | | | |
|---|---|---|---|---|---|---|---|---|---|
| | Bsp. 1 | Bsp. 2 | Bsp. 3 | Bsp. 4 | Bsp. 5 | Bsp. 6 | Bsp. 7 | Bsp. 8 | Bsp. 9 |
| Caprylic/Capric Triglyceride | 8 | 5 | 5 | 8 | 4,5 | 5 | 8 | 6 | 5 |
| Cetyl Alkohol | 3 | 3 | 3 | 3 | 3 | 3 | 3 | 3 | 2,5 |
| Methylglucose Sesquistearate | 3 | 4 | | 3 | 4 | 1 | 3 | 3 | |
| Tocopheryl Acetate | 1 | | 1 | 1 | | 1 | 1 | | 1 |
| Hydrogenatet Coco Glycerides | 1 | 1 | 1 | 1 | 1 | 1 | 1 | 1 | 1 |
| Cetyl Palmitate | 1 | 1 | 1 | 1 | 1 | 1 | 1 | 1 | 1 |
| Potassium Cetyl Phosphate, Hydrogenated Palm Glycerides | | | 3 | | 1 | 2,5 | | | 3 |
| Isodecyl Neopentanoate | | | 3 | | 1 | 2,5 | | | |
| Isodecyl Neopentanoate, Diisopropyl Sebacate, Lauryl Lactate | | | 3 | | 1 | 2 | | | |
| Carbomer (Carbopol 981) | 0,2 | 0,2 | 0,2 | 0,2 | 0,2 | 0,2 | 0,2 | 0,2 | 0,2 |
| Xanthan Gum | 0,1 | 0,2 | 0,2 | 0,1 | 0,2 | 0,2 | 0,1 | 0,2 | 0,2 |
| Glycerin | 4 | 4 | 4 | 4 | 4 | 4 | 4 | 4 | 4 |
| Panthenol | 1 | 1 | 1 | 1 | 1 | 1 | 1 | 1 | 1 |
| Disodium EDTA | 0,1 1 | 0,1 | 0,1 | 0,1 | 0,1 | 0,1 | 0,1 | 0,1 | 0,1 |
| Sodium Hydroxide | 0,05 | 0,05 | 0,05 | 0,05 | 0,05 | 0,05 | 0,05 | 0,05 | 0,05 |
| Alkohol denat. | 9 | 9 | 9 | 9 | 9 | 9 | 9 | 9 | 9 |
| Benzotriazolderivat gem. Formel VI | 5 | 3 | 2,5 | 1 | 2 | 8 | 3 | 2,5 | 4 |
| Verbindung gem, Formel IIa | 0,8 | | | 0,2 | 0,6 | | | 1,2 | 0,4 |
| Verbindung gem. Formel IIb | | 0,6 | 0,3 | | | 0,6 | | 0,2 | |
| Verbindung gem. Formel IIc | | | 0,2 | 0,2 | | 0,2 | 1 | | 0,4 |
| Verbindung gem. Formel IId | | | 0,25 | | | | | | |
| Ethylhexyl Triazone (Uvinul T 150) | 5 | | 1,5 | 3 | 3 | | 4 | 4 | |
| Methylene Bis-Benzotriazolyl Tetramethylbutylphenol (Tinosorb M) | | | 11 | 2 | | 4 | | 2 | 2 |
| Bis-Ethylhexyloxyphenol Methoxyphenyl triazine (Tinosarb S) | | 2,5 | 2 | | | | 1 | | |
| Diethylamino Hydroxybenzoyl Hexyl Benzoate (Uvinul A Plus) | 5 | 5 | | | | 3 | | 2 | 4 |
| Ethylhexyl Bis-Isopentylbenzoxazolylphenyl Melamine (Uvasorb K2A) | | | | | 2 | | 0,5 | | |
| Phenylbenzimidazol Sulfonic Acid (Neo Heliopan Hydro) | | | | 4 | | 2 | | | |
| Octocrylene (Uvinul N 539) | | 3 | | | | 3 | 2 | 3 | |
| Ethylhexyl Methoxycinnamate (Neo Heliopan AV) | | | | 2 | | | 1 | | |
| Isoamyl-p-Methoxycinnamate (Neo Heliopan E 1000) | 5 | 5 | | | 4 | 6 | | | |
| Diethylhexyl Butamido Triazone (Uvasorb HEB) | | 2,5 | | | 2 | 1 | 1 | | 2 |
| Ethylhexyl Salicylate (Neo Heliopan OS) | 4 | 4 | | | | | 2 | | 2 |
| Butyl Methoxydibenzoylmethane (Parsol 1789) | | | | 2 | 1 | | | 4 | 1 |
| Titaniumdioxide | | | | 2 | | | | | |
| Aqua | ad 100 | ad 100 | ad 100 | ad 100 | ad 100 | ad 100 | ad 100 | ad 100 | ad 100 |

### 0/W-Emulsionen:

| Inhaltsstoffe | Gehalt [Gew.-%] | | | | | | | | |
|---|---|---|---|---|---|---|---|---|---|
| | Bsp. 10 | Bsp. 11 | Bsp. 12 | Bsp. 13 | Bsp. 14 | Bsp. 15 | Bsp. 16 | Bsp. 17 | Bsp. 18 |
| Caprylic/Capric Triglyceride | | 0,2 | | | | | | 0,4 | |
| Cetyl Alkohol | 1,5 | 1,5 | 1,2 | 1,5 | 1,8 | 1,2 | 1,5 | 1,5 | 1,2 |
| Methylglucose Sesquistearate | 2 | 1,8 | 2 | 2 | 2 | 2 | 2 | 1,8 | 2 |
| Tocopheryl Acetate | 1 | 1 | 0.8 | 1 | 1,2 | 0.8 | 1 | 1 | 0.8 |
| Hydrogenatet Coco Glycerides | 0,5 | 0,5 | 0,8 | 0,5 | 0,8 | 0,8 | 0,6 | 0,5 | 0,8 |
| Coco Glycerides | 5 | 5 | 4 | 5 | 4,5 | 4 | 4 | 5 | 4 |
| Cetyl Palmitate | 0,5 | 0,5 | 0,5 | 0,5 | 0,5 | 0,5 | 0,5 | 0,5 | 0,5 |
| Xanthan Gum | 0,1 | 0,1 | 0,1 | 0,1 | 0,1 | 0,1 | 0,1 | 0,1 | 0,1 |
| Disodium EDTA | 0,1 | 0,1 | 0,1 | 0,1 | 0,1 | 0,1 | 0,1 | 0,1 | 0,1 |
| Sodium Carborner | 0,2 | 0,2 | 0,2 | 0,2 | 0,2 | 0,2 | 0,2 | 0,2 | 0,2 |
| Phenoxyethanol | 1 | 1 | 1 | 1 | 1 | 1 | 1 | 1 | 1 |
| Benzotriazolderivat gem. Formel VI | 8 | 4 | 1 | 5 | 5 | 2 | 0,5 | 10 | 3 |
| Verbindung gem. Formel IIa | 0,8 | 0,4 | 0,2 | 0,6 | 0,2 | | | 1,2 | |
| Verbindung gem. Formel IIb | 0,1 | | | | 0,2 | | | | |
| Verbindung gem. Formel IIc | 0,2 | | 0,2 | | | | 0,3 | | |
| Verbindung gem. Formel IId | | | | 0,2 | | 0,5 | | | 0,5 |
| Ethylhexyl Triazone (Uvinul T 150) | 5 | | | | 4 | | | 2 | |
| Diethylamino Hydroxybenzol Hexyl Benzoate (Uvinul A Plus) | 1 | | 3 | | | | 5 | 2 | |
| Octocrylene (Uvinul N 539) | 2 | | | | 3 | | | | |
| Ethylhexyl Methoxycinnamate (Neo Heliopan AV) | | 2 | | | | | | | 4 |
| Isoamyl-p- Methoxycinnamate (Neo Heliopan E 1000) | | 6 | | | | | | | |
| Diethylhexyl Butamido Triazone (Uvasorb HEB) | | | 2 | | | 2 | | | |
| Titaniumdioxide | | | | | | 1 | | 3 | |
| Aqua | ad 100 | ad 100 | ad 100 | ad 100 | ad 100 | ad 100 | ad 100 | ad 100 | ad 100 |

### II. W/O-Emulsionen:

| Inhaltsstoffe | Gehalt [Gew.-%] | | | | | | | | |
|---|---|---|---|---|---|---|---|---|---|
| | Bsp.1 | Bsp. 2 | Bsp. 3 | Bsp. 4 | Bsp. 5 | Bsp. 6 | Bsp. 7 | Bsp. 8 | Bsp. 9 |
| Caprylic/Capric Triglyceride | 6 | 6 | 6 | 6 | 5 | 5 | 5 | 5 | 5 |
| Isopropylmyristate | 10 | 10 | 10 | 10 | 8 | 8 | 8 | 8 | 8 |
| Polyglyceryl-2-Dipolyhydroxystearate | 3 | 3 | 3 | 3 | 4 | 4 | 4 | 4 | 4 |
| Tocopheryl Acetate | 1 | 1 | 1 | 1 | 1 | 1 | 1 | 1 | 1 |
| Cera Alba | 1 | 1 | 1 | 1 | 1 | 1 | 1 | 1 | 1 |
| Glyceryl Oleate | 1 | 1 | 1 | 1 | 1 | 1 | 1 | 1 | 1 |
| Disodium EDTA | 0,1 | 0,1 | 0,1 | 0,1 | 0,1 | 0,1 | 0,1 | 0,1 | 0,1 |
| Bisabolol | 0,1 | 0,1 | 0,1 | 0,1 | 0,1 | 0,1 | 0,1 | 0,1 | 0,1 |
| Magnesium Sulfate | 0,5 | 0,5 | 0,5 | 0,5 | 0,5 | 0,5 | 0,5 | 0,5 | 0,5 |
| Magnesium Stearate | 0,5 | 0,5 | 0,5 | 0,5 | 0,5 | 0,5 | 0,5 | 0,5 | 0,5 |
| NaOH | | | | | | 0,3 | 0,5 | | |
| Alkohol denat. | 7 | 7 | 7 | 7 | 7 | 7 | 7 | 7 | 7 |
| Benzotriazolderivat gem. Formel VI | 8 | 6 | 4 | 3 | 3 | 4 | 2 | 1 | 2 |
| Verbindung gem. Formel IIa | | | 0,2 | 0,6 | | 0,3 | | 0,2 | 0,3 |
| Verbindung gem. Formel IIb | 0,8 | 0,4 | 0,2 | | | | 0,6 | | 0,3 |
| Verbindung gem. Formel IIc | 0,2 | | 0,2 | | 0,4 | 0,3 | | | |
| Verbindung gem. Formel IId | | 0,4 | 0,2 | | 0,2 | | | 0,2 | |
| Ethylhexyl Triazone (Uvinul T 150) | 4 | 4 | 2 | 5 | | | | | |
| Methylene Bis-Benzotriazolyl Tetramethylbutylphenol | 2 | | 2 | | | | | | |
| Bis-Ethylhexyloxyphenol Methoxyphenyl triazine (Tinosorb S) | 2 | | | | 4 | 4 | | | |
| Diethylamino Hydroxybenzoyl Hexyl Benzoate (Uvinul A Plus) | 2 | 1 | | | | 4 | | | 4 |
| Ethylhexyl Bis-Isopentylbenzoxazolylphenyl Melamine (Uvasorb K2A) | | | 2 | | 3 | | | | |
| Phenylbenzimidazol Sulfonic Acid (Neo Heliopan Hydro) | | | | | | 5 | 5 | | |
| Octocrylene (Uvinul N 539) | | | 3 | | | 2 | | | 2 |
| Ethylhexyl Methoxycinnamate (Neo Heliopan AV) | | 2 | | | | | | | |
| Isoamyl-p-Methoxycinnamate (Neo Heliopan E 1000) | | | | | | | | 4 | |
| Diethylhexyl Butamido Triazone (Uvasorb HEB) | | | | | | 4 | 4 | 2 | |
| Ethylhexyl Salicylate (Neo Heliopan OS) | 4 | | | 3 | | | | 2 | |
| Butyl Methoxy-dibenzoylmethane (Parsol 1789) | | 2 | | | 2 | | | 5 | |
| Titaniumdioxide | | 2 | | | 3 | | 4 | | |
| Aqua | ad 100 | ad 100 | ad 100 | ad 100 | ad 100 | ad 100 | ad 100 | ad 100 | ad 100 |

### W/O-Emulsionen:

| Inhaltsstoffe | Gehalt [Gew.-%] | | | | | | | | |
|---|---|---|---|---|---|---|---|---|---|
| | Bsp. 10 | Bsp. 11 | Bsp. 12 | Bsp. 13 | Bsp. 14 | Bsp. 15 | Bsp. 16 | Bsp. 17 | Bsp. 18 |
| Caprylic/Capric Triglyceride | 10 | 10 | 10 | 10 | 10 | 10 | 10 | 10 | 10 |
| Polyglyceryl-4, Diisostearate/ Polyhydroxystearate/ Sebacate | 4 | 4 | 4 | 4 | 4 | 4 | 4 | 4 | 4 |
| C12-15 Alkyl Benzoate | 10 | 10 | 10 | 10 | 10 | 10 | 10 | 10 | 10 |
| Tocopheryl Acetate | 0,5 | 0,5 | 0,5 | 0,5 | 0,5 | 0,5 | 0,5 | 0,5 | 0,5 |
| Butylenglykol | 5 | 5 | 5 | 5 | 5 | 5 | 5 | 5 | 5 |
| Cera Alba | 1 | 1 | 1 | 1 | 1 | 1 | 1 | 1 | 1 |
| Glycine | 1 | 1 | 1 | 1 | 1 | 1 | 1 | 1 | 1 |
| Trisodium NTA,aqua | 0,26 | 0,26 | 0,26 | 0,26 | 0,26 | 0,26 | 0,26 | 0,26 | 0,26 |
| Citic Acid | 0,06 | 0,06 | 0,06 | 0,06 | 0,06 | 0,06 | 0,06 | 0,06 | 0,06 |
| Magnesium Sulfate | 0,5 | 0,5 | 0,5 | 0,5 | 0,5 | 0,5 | 0,5 | 0,5 | 0,5 |
| Benzotriazolderivat gem. Formel VI | 2 | 5 | 6 | 3 | 1 | 2 | 3 | 2,5 | 4 |
| Verbindung gem. Formel IIa. | | | 0,25 | 0,4 | | 0,2 | 0,3 | | 0,2 |
| Verbindung gem. Formel IIb | | | 0,3 | | | 0,3 | | 0,4 | |
| Verbindung gem. Formel IIc | 0,2 | | | | 0,3 | 0,3 | 0,1 | | |
| Verbindung gem. Formel IId | | 0,4 | | | | 0,2 | | | 0,8 |
| Ethylhexyl Triazone (Uvinul T 150) | | 5 | | 3 | | | 4 | | |
| Methylene Bis-Benzotriazolyl Tetramethylbutylphenol | | 4 | | | | 2 | | | |
| Bis-Ethylhexyloxyphenol Methoxyphenyl triazine (Tinosorb S) | 2 | | 4 | | | | 4 | | |
| Diethylamino Hydroxybenzoyl Hexyl Benzoate (Uvinul A Plus) | 5 | | 1 | | | 5 | 3 | | 5 |
| Ethylhexyl Bis-Isopentylbenzoxazolylphenyl Melamine (Uvasorb K2A) | | 2 | | | | | 1 | | |
| Octocrylene (Uvinul N 539) | 5 | | | | 5 | | | 2 | |
| Ethylhexyl Methoxycinnamate (Neo Heliopan AV) | | | 2 | | | | | | |
| Diethylhexyl Butamido Triazone (Uvasorb HEB) | | | | | 4 | 3 | | | |
| Ethylhexyl Salicylate (Neo Heliopan OS) | 5 | | | | | | | | |
| ButylMethoxy- dibenzoylmethane (Parsol 1789) | 5 | | | 2 | | 4 | | | |
| Titaniumdioxide | 4 | | | | | 2 | | | |
| Aqua | ad 100 | ad 100 | ad 100 | ad 100 | ad 100 | ad 100 | ad 100 | ad 100 | ad 100 |

### III. Hydrodispersionsgele:

| Inhaltsstoffe | Gehalt [Gew.-%] | | | | | | | | |
|---|---|---|---|---|---|---|---|---|---|
| | Bsp. 1 | Bsp. 2 | Bsp. 3 | Bsp. 4 | Bsp. 5 | Bsp. 6 | Bsp. 7 | Bsp. 8 | Bsp. 9 |
| Arachis Hypogaea | 5 | 4 | 5 | 4 | 5 | 5 | 5 | 4 | 5 |
| Octyldodecanol | 5 | 4,5 | 5 | 4,5 | 5 | 5,5 | 5 | 4,5 | 5 |
| Caprylic/Capric Triglyceride | 5 | 6 | 5 | 6 | 5 | 5 | 5 | 6 | 5 |
| Tocopheryl Acetate | 1 | 1 | 1 | 1 | 1 | 1 | 1 | 1 | 1 |
| Phenoxyethanol | 1 | 1 | 1 | 1 | 1 | 1 | 1 | 1 | 1 |
| Acrylates/C10-30 Alkyl Acrylate Crosspolymer-Sodium | 0,5 | 0,5 | 0,5 | 0,5 | 0,5 | 0,5 | 0,5 | 0,5 | 0,5 |
| Disodium EDTA | 0,1 | 0,1 | 0,1 | 0,1 | 0,1 | 0,1 | 0,1 | 0,1 | 0,1 |
| Benzotriazolderivat gem. Formel VI | 2 | 2 | 4 | 4 | 6 | 6 | 2 | 2 | 1 |
| Verbindung gem. Formel IIa | | 0,2 | 0,8 | | | 1 | | | 0,2 |
| Verbindung gem. Formel IIb | | 0,4 | | 0,3 | 0,4 | 0,2 | 0,5 | | |
| Verbindung gem. Formel IIc | | 0,2 | 0,5 | | 0,4 | | | 0,4 | |
| Verbindung gem. Formel IId | 0,6 | | | 0,5 | | | | | 0,2 |
| Ethylhexyl Triazone (Uvinul T 150) | | | | | | 5 | 5 | 3 | 3 |
| Bis-Ethylhexyloxyphenol Methoxyphenyl triazine (Tinosorb S) | 2 | 4 | | | | 2 | 2 | | |
| Diethylamino Hydroxybenzoyl Hexyl Benzoate (Uvinul A Plus) | | 2 | | | | | | 4 | |
| Ethylhexyl Bis-Isopentylbenzoxazolylphenyl Melamine (Uvasorb K2A) | | 1 | | | 2 | 2 | | | |
| Phonylbenzimidazol Sulfonic Acid (Neo Heliopan Hydro) | | 5 | | 3 | | | | 5 | |
| Octocrylene (Uvinul N 539) | 2 | | | 2 | 2 | | | | |
| Ethylhexyl Methoxycinnamate (Neo Heliopan AV) | 2 | | | | | | | | |
| Isoamyl-p-Methoxycinnamate (Neo Heliopan E 1000) | | 4 | | | | 4 | | | |
| Diethylhexyl Butamido Triazone (Uvasorb HEB) | | 4 | 4 | 4 | | | | | |
| Ethylhexyl Salicylate (Neo Heliopan OS) | | | | | 3 | | | | |
| Butyl Methoxy-dibenzoylmethane (Parsol 1789) | | | 2 | 4 | 2 | | | | |
| Titaniumdioxide | | 2 | | | | 4 | | | |
| Aqua | ad 100 | ad 100 | ad 100 | ad 100 | ad 100 | ad 100 | ad 100 | ad 100 | ad 100 |

### Hydrodispersionsgele:

| Inhaltsstoffe | Gehalt [Gew.-%] | | | | | | | | |
|---|---|---|---|---|---|---|---|---|---|
| | Bsp. 10 | Bsp. 11 | Bsp. 12 | Bsp. 13 | Bsp. 14 | Bsp. 15 | Bsp. 16 | Bsp. 17 | Bsp. 18 |
| Tocopherol | 1 | 1 | 1 | 1 | 1 | 1 | 1 | 1 | 1 |
| Acrylates/C10-30 Alkyl Acrylate Crosspolymer-Sodium | 0,5 | 0,5 | 0,5 | 0,5 | 0,5 | 0,5 | 0,5 | 0,5 | 0,5 |
| Disodium EDTA | 0,1 | 0,1 | 0,1 | 0,1 | 0,1 | 0,1 | 0,1 | 0,1 | 0,1 |
| Benzotriazolderivat gem. Formel VI | 2 | 2 | 2 | 2 | 4 | 4 | 4 | 4 | 8 |
| Verbindung gem. Formel IIa | 0,1 | | | 0,4 | | | 0,8 | | 0,2 |
| Verbindung gem. Formel IIb | | 0,2 | | 0,2 | | 0,2 | | 0,4 | |
| Verbindung gem. Formel IIc | 0,2 | 0,2 | | | 0,2 | 0,2 | | | 0,5 |
| Verbindung gem. Formel IId | | | 0,6 | | | 0,2 | | | |
| Ethylhexyl Triazone (Uvinul T 150) | 4 | | | 4 | | 2 | 2 | | |
| Methylene Bis-Benzotriazolyl Tetramethylbutylphenol (Tinosorb M) | 8 | | 10 | | | | | 14 | 8 |
| Bis-Ethylhexyloxyphenol Methoxyphenyl triazine (Tinosorb S) | | 2 | | | | | | | |
| Diethylamino Hydroxybenzoyl Hexyl Benzoate (Uvinul A Plus) | 2 | 3 | | | | 4 | | | |
| Ethylhexyl Bis-Isopentylbenzoxazolylphenyl Melamine (Uvasorb K2A) | 2 | | | | 2 | | | | |
| Phenylbenzimidazol Sulfonic Acid (Neo Heliopan Hydro) | | | | | | 5 | | | |
| Octocrylene (Uvinul N 539) | | 3 | | | | | | | |
| Ethylhexyl Methoxycinnamate (Neo Heliopan AV) | | 1 | | | | | | | |
| Isoamyl-p-Methoxycinnamate (Neo Heliopan E 1000) | | | | | | 5 | | | |
| Diethylhexyl Butamido Triazone (Uvasorb HEB) | | | 4 | 4 | | | | | |
| Ethylhexyl Salicylate (Neo Heliopan OS) | 2 | | | | | | 2 | | |
| Butyl Methoxy-dibenzoylmethane (Parsol 1789) | | 3 | | | 3 | | | | |
| Titaniumdioxide | | | 2 | | | 4 | | 1 | |
| Aqua | ad 100 | ad 100 | ad 100 | ad 100 | ad 100 | ad 100 | ad 100 | ad 100 | ad100 |

## Patentansprüche

1. Kosmetische Zusammensetzung zur topischen Anwendung, insbesondere zum Lichtschutz der Haut und/oder der Haare,
**dadurch gekennzeichnet, dass** sie in einem kosmetisch akzeptablen Träger enthält:
i) ein Benzotriazolderivat der Formel 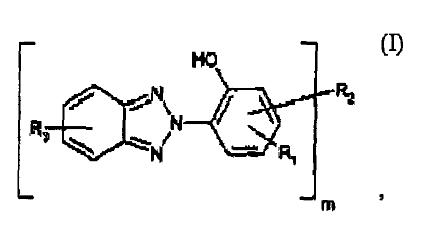 wobei
R₁ C₁-C₃₀-Alkyl, C₁-C₅-Alkoxy, C₁-C₅-Alkoxycarbonyl, C₅-C₇-Cycloalkyl, C₆-C₁₀-Aryl, Arylalkyl, -SO₃M oder ein Radikal der Formel 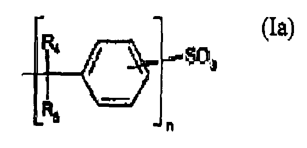 ist;
R₃ Wasserstoff, C₁-C₅-Alkyl, C₁-C₅-Alkoxy, Halogen, vorzugsweise Cl oder Hydroxy ist;
R₄ und R₅ voneinander unabhängig Wasserstoff oder C₁-C₅-Alkyl sind;
m 1 oder 2 ist;
n 0 oder 1 ist;
wenn m = 1 ist, R₂ Wasserstoff, unsubstituiertes oder phenylsubstituiertes
C₁-C₁₂-Alkyl oder C₆-C₁₀-Aryl ist;
wenn m = 2 ist, R₂ eine Bindung oder -(CH₂)ₚ ist und p 1, 2 oder 3 ist und
ii) eine Verbindung der Formel 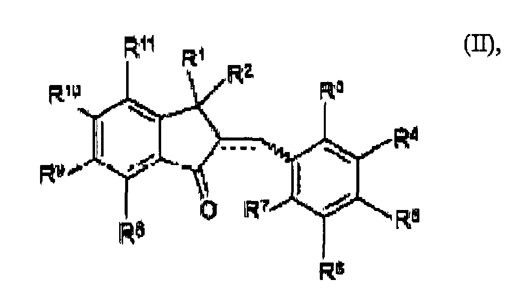
wobei
R¹ und R² unabhängig voneinander Wasserstoff oder C₁-C₁₂-Alkyl sind;
R³ bis R¹¹ unabhängig voneinander Wasserstoff, C₁-C₁₂-Alkyl, Hydroxy oder C₁-C₁₂-Alkoxy sind
und die gestrichelte Linie eine Doppelbindung oder zwei Wasserstoffatome darstellt.

2. Zusammensetzung nach Anspruch 1,
**dadurch gekennzeichnet, dass** das Benzotriazolderivat gemäß i) ein Benzotriazolderivat der Formel 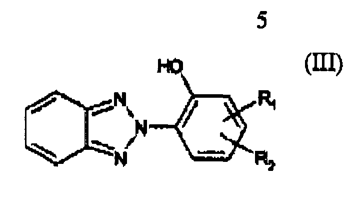 ist, wobei
R₁ C₁-C₃₀-Alkyl ist; und
R₂ Wasserstoff oder C₁-C₁₂-Alkyl ist.

3. Zusammensetzung nach Anspruch 1 oder 2,
**dadurch gekennzeichnet, dass** das Benzotriazolderivat ein Benzotriazolderivat der Formel 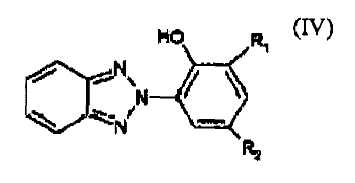 ist, wobei
R₁ eine Mischung aus wenigstens drei isomeren, verzweigten sekundären Alkylgruppen mit je 8-30 Kohlenstoffatomen der Formel 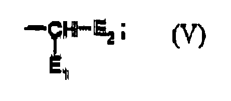 ist, wobei
E₁ geradkettiges C₁-C₁₄-Alkyl ist;
E₂ geradkettiges C₄-C₁₅-Alkyl ist;
wobei die Gesamtzahl an Kohlenstoffatomen in E₁ plus E₂ 7 bis 29 beträgt; und
R₂ C₁-C₅-Alkyl ist.

4. Zusammensetzung nach einem der Ansprüche 1 bis 3, wobei R₁ C₈-C₁₅-Alkyl ist.

5. Zusammensetzung nach einem der Ansprüche 1 bis 4, wobei das Benzotriazolderivat das Benzotriazol der Formel 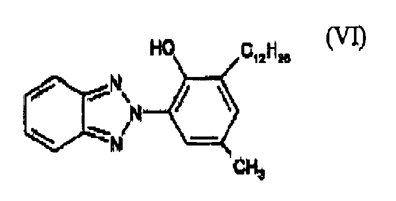 ist.

6. Zusammensetzung nach einem der Ansprüche 1 bis 5, wobei in der Verbindung der Formel II gem. ii)
R¹ und R² unabhängig voneinander C₁-C₁₂-Alkyl sind.

7. Zusammensetzung nach einem der Ansprüche 1 bis 6, wobei in der Verbindung der Formel II gem. ii)
R¹ und R² Methyl sind.

8. Zusammensetzung nach einem der Ansprüche 1 bis 7, wobei die Verbindung der Formel II gem. ii) eine Verbindung der Formeln IIa, IIb, IIc oder IId ist:

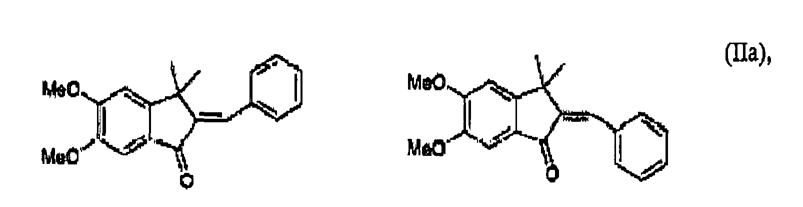

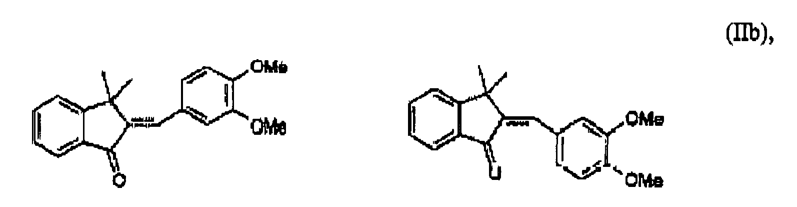

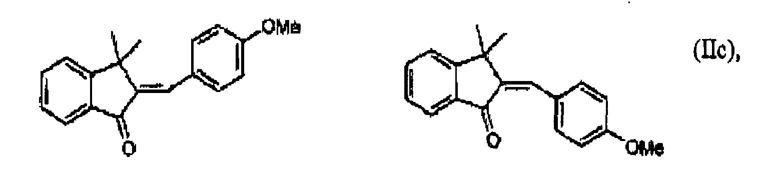

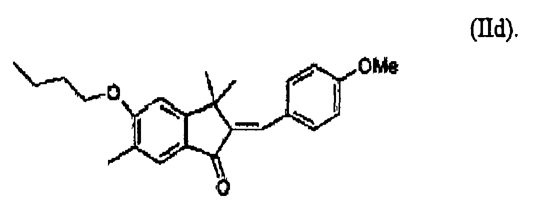

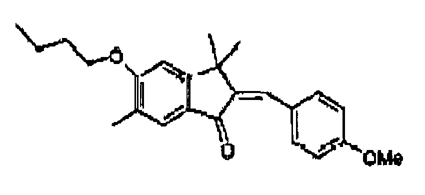

9. Zusammensetzung nach einem der Ansprüche 1 bis 8,
**dadurch gekennzeichnet, dass** das Benzotriazolderivat gem. i) das Benzotriazolderivat der Formel VI ist und die Verbindung gem. ii) eine Verbindung der Formeln IIa, IIb, IIc oder IId ist.

10. Zusammensetzung gemäß einem der Ansprüche 1 bis 9,
**dadurch gekennzeichnet, dass** das Benzotriazolderivat in einem Anteil von 0,1 bis 20 Gew,-%, bezogen auf das Gesamtgewicht der Zusammensetzung, vorliegt.

11. Zusammensetzung gemäß einem der Ansprüche 1 bis 10,
**dadurch gekennzeichnet, dass** die Verbindung der Formel II, insbesondere der Formeln IIa, IIb, IIc oder IId in einem Anteil von 0,0001 bis 10 Gew.-%, bezogen auf das Gesamtgewicht der Zusammensetzung, vorliegt.

12. Zusammensetzung nach einem der Ansprüche 1 bis 11,
**dadurch gekennzeichnet, dass** der kosmetisch akzeptable Träger als Lotion, Creme, Emulsion, Salbe, Gel, Puder oder Suspension formuliert ist.

13. Zusammensetzung nach einem der Ansprüche 1 bis 12,
**dadurch gekennzeichnet, dass** sie zusätzlich mindestens einen UV-Filter, ausgewählt aus
p-Aminobenzoesäurederivaten, z.B. 4-Aminobenzoesäure, 4-Dimethylaminobenzoesäure-2-ethylhexylester oder ethoxyliertes Ethyl-4-aminobenzoat,
Salicylsäurederivaten, z.B. 3,3,5-Trimethyl-cyclohexyl-salicylat oder Salicylsäure-2-ethylhexylester,
Benzophenonderivaten, z.B. 2-Hydroxy-4-methoxy-benzophenon, 2-Hydroxy-4-methoxy-benzophenon-5-sulfonsäure und ihr Natriumsalz oder 2-[4-(Diethylamino)-2-hydroxybenzoyl]-Benzoesäurehexylester,
Sulfonsäurederivaten, z.B. 2-Phenylbenzimidazol-5-sulfonsäure und ihre Kalium-, Natrium- und Triethanolaminsalze oder Benzol-1,4-di(3-methyliden-10-camphersulfonsäure),
Dibenzoylmethanderivaten, z.B. 1-(4-tert-Butylphenyl)-3-(4-methoxyphenyl)propan-1,3-dion,
Diphenylacrylaten, z.B. 2-Cyano-3,3-diphenylacrylsäure-2-ethylhexylester,
Mothoxyzimtsäurederivaten, z.B. 4-Methoxyzimtsäure-2-ethylhexylester oder 4-Methoxy-zimtsäureisoamylester,
Campherderivaten, z.B. 3-(4'-Trimethylammonium)-benzyliden-boman-2-on methylsulfat, 3-(4'-sulfo)-benzyliden-bornan-2-on und seine Salze oder 3-(4'-Methylbenzyliden)-DL-campher oder 3-Benzylidencampher,
Triazinderivaten, z.B. 2,4,6-Tris[p-(2-ethylhexyl-oxycarbonyl)amilino] 1,3,5-triazin, 4,4'-[(6-[4-((1,1-Dimethylethyl)aminocarbonyl)phenyl-amino]-1,3,5-triazin-2,4-diyl)diinono]bis(benzoesäure-2-ethylhexylester) oder 2,4-Bis[4-(2-ethylhexyloxy)-2-hydroxyphenyl]-6-(4-methoxyphenyl)-1,3,5-triazin,
Benzotriazolderivaten, z.B. 2,2'-Methylen-bis(6-(2H-benzotriazol-2-yl)-4-(1,1,3,3-tetramethylbutyl)phenol) oder 2-(2H-Benzotriazol-2-yl)-4-methyl-6-(2-methyl-3-(1,3,3,3-tetramethyl-1-(trimethylsilyloxy) disiloxanyl)propyl)phenol,
Benzimidazolderivaten, z.B. 2,2'-(1,4-Phenylen)bis(1H-benzimidazol-4,6-disulfonsäure, Mononatriumsalz),
Benzoxazol-Derivaten, z.B. 2,4-Bis-[5-1(dimethylpropyl)benzoxazol-2-yl-(4-phenyl)-imino]-6-(2-ethylhexyl)-imino-1,3,5-triazin,
Polymer von N-[2(und 4)-(2-oxoborn-3-ylidenmethyl)benzyl]acrylamid,
3-(4-(2,2-bis Ethoxycarbonylvinyl)-phenoxy)propenyl)-methoxysiloxan/Dimethylsiloxan-copolymer,
Pigmenten oder Nanopigmenten von Metalloxiden, die ggf. ummantelt sind, z.B. Oxide von Titan, Zink, Eisen, Zirkonium oder Cer sowie deren Gemische, enthält,
